# EUROPEAN PATENT SPECIFICATION

(11) **EP 1 879 862 B1**
(45) Date of publication and mention of the grant of the patent: **19.01.2011**
(21) Application number: 06765662.9
(22) Date of filing: 03.05.2006
(51) Int. Cl.: C07D 207/34, A61P 3/00, A61K 31/40

(54) **MAGNESIUM SALTS OF HMG-COA REDUCTASE INHIBITORS**
MAGNESIUMSALZE VON HMG-COA-REDUKTASE-HEMMERN
SELS DE MAGNESIUM D'INHIBITEURS DE HMG-COA REDUCTASE

(30) Priority: 03.05.2005 IN DE11082005
(43) Date of publication of application: 23.01.2008
(60) Divisional application: 08161846.4
(73) Proprietor: Ranbaxy Laboratories Limited, Gurgaon, Haryana 122001 (IN)
(72) Inventor: KUMAR, Yatendra, Gurgaon, Haryana 122001 (IN); KUMAR, Saridi Madhava Dileep, Vizianagaram, Andhra Pradesh 535102 (IN); SATHYANARAYANA, Swargam, Karim Nager, Andhra Pradesh 505002 (IN)
(74) Representative: Cronin, Brian Harold John
(86) International application number: PCT/IB2006/051396
(87) International publication number: WO 2006/117761

(56) References cited:
- EP-A- 1 336 405
- WO-A-2006/070248
- WO-A2-2006/054308
- WO-A2-2006/084474

## Description

### Field of the Invention

The present invention relates to crystalline magnesium salts of atorvastatin of structural formula I, processes for their preparation, pharmaceutical compositions thereof and compositions for treating mammals suffering from hypercholesterolemia.

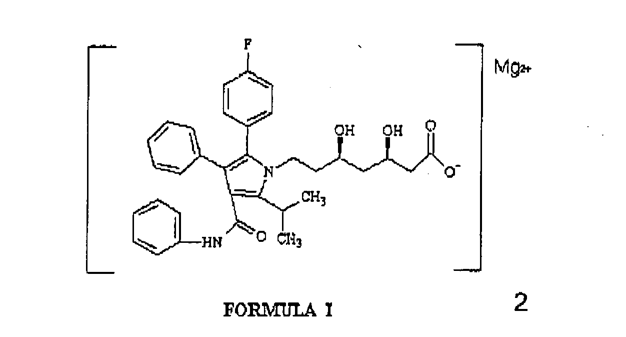

### Background of the Invention

Atorvastatin is represented by Formula II and is a member of the class of drugs called statins. Statins are currently the most therapeutically effective drugs available for reducing low-density lipoprotein (LDL) particle concentration in the blood stream of patients at risk for cardiovascular disease.

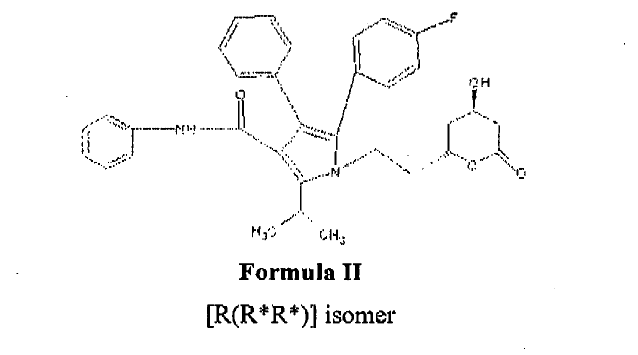

Statins' mechanism of action of has been elucidated in some detail. They interfere with the synthesis of cholesterol and other sterols in the liver by competitively inhibiting the 3-hydroxy-3-methyl-glutaryl-coenzyme A reductase enzyme ("HMG-CoA reductase"). HMG-CoA reductase catalyses the conversion of HMG to mevalonate, which is the rate determining step in the biosynthesis of cholesterol. This inhibition leads to a reduction in the concentration of cholesterol in the liver.

EP 1 336 405 describes amorphous atorvastatin including amorphous atorvastatin magnesium. It also describes amorphous atorvastatin of a particular particle size and the stabilization of atorvastatin with an alkali salt metal additive. Crystalline atorvastatin is referred to in terms of the marketed formulation of Lipitor™), which is crystalline atorvastatin calcium.

WO 2006/054308 (published 26 May 2006) discloses an atorvastatin salt of calcium or magnesium, that can be amorphous or crystalline, specifically the crystalline form (VI) of atorvastatin calcium.

WO 2006/084474 (published 17 August 2006) relates to a pharmaceutical composition containing an HMG-CoA reductase inhibitor from a large group comprising atorvastatin. Crystalline atorvastatin magnesium is mentioned without providing any details about its characterization or preparation.

WO 2006/070248 (published 6 July 2006) relates to the blending of atorvastatin and amlodipine. It states in general terms that atorvastatin, including atorvastatin magnesium, may exist in any of the solid state forms available such as amorphous, crystalline or any other polymeric form.

### Summary of the Invention

The invention provides a crystalline form of magnesium salt of atorvastatin having
(a) an XRD pattern comprising peaks at 2Θ values of about 8.60, 16.14, 17.96 and 21.44±0.2, and/or
(b) an IR spectrum comprising absorption bands at about 510.0, 525.0, 614.3, 692.0, 716.4, 755.9, 811.9, 847.4, 885.9, 916.9, 970.6, 1014.0, 1031.7, 1076.2, 1092.0, 1110.7, 1155.4, 1222.5, 1313.0, 1435.8, 1506.1, 1524.4, 1594.4, 1654.4, 1898.2, 1947.3, 2954.3, 3283.6, 3405.8, and 3667.3 cm-'.

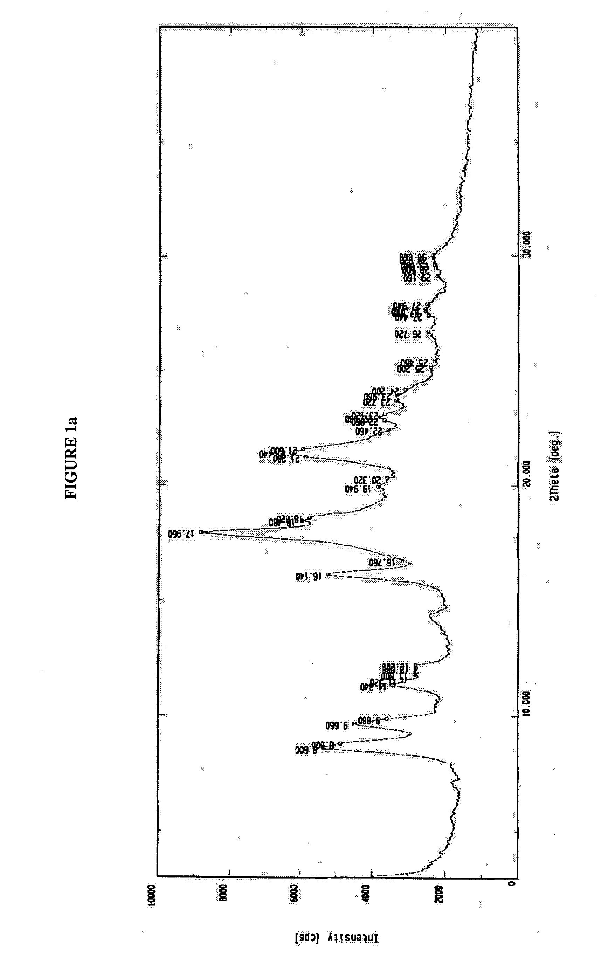

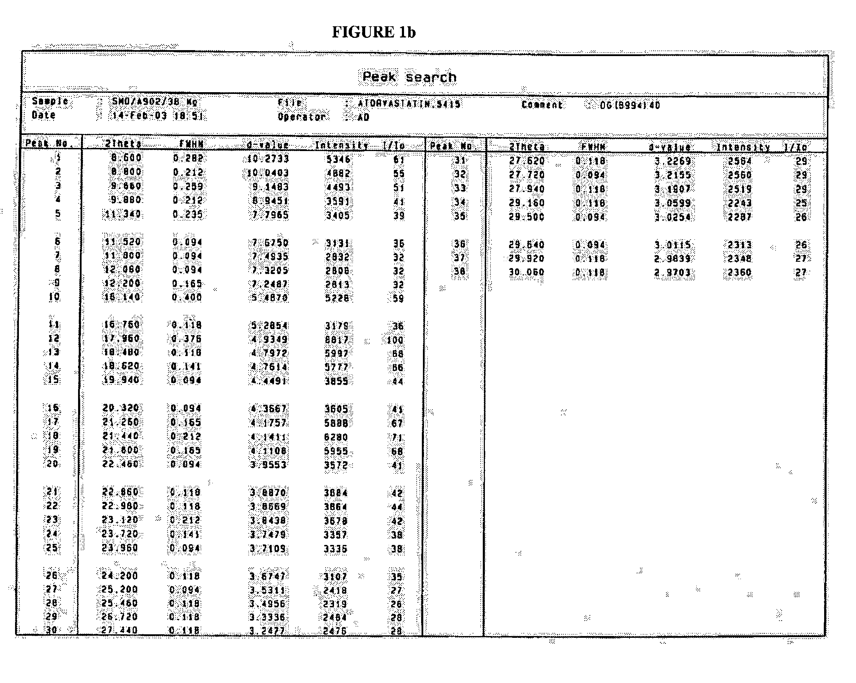

The invention thus provides a crystalline form of a magnesium salt of atorvastatin having XRD pattern essentially as shown in Figure 1.

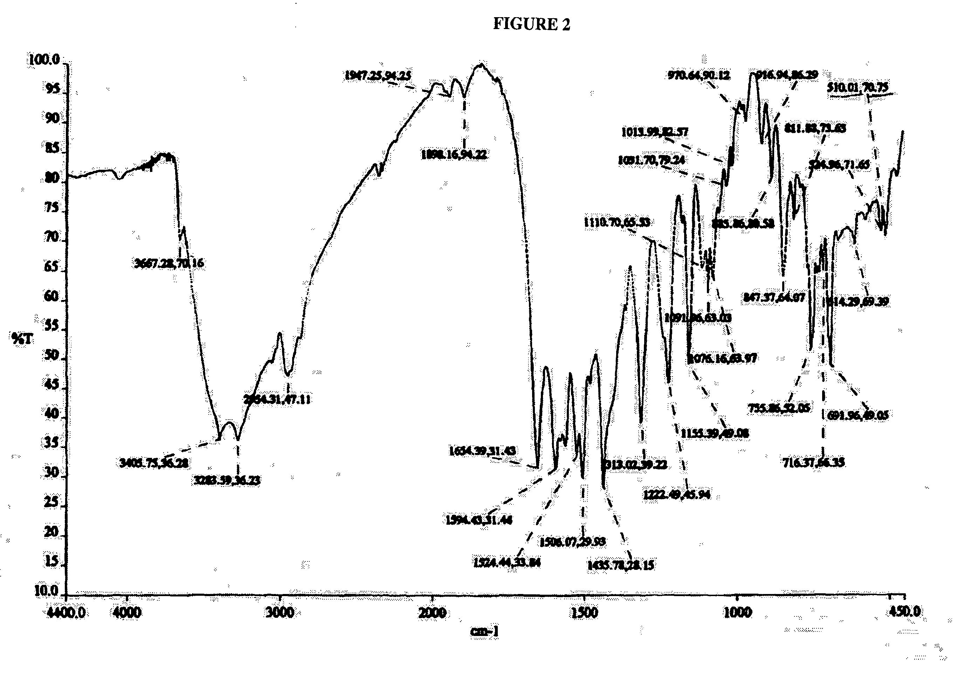

The invention also provides a crystalline form of a magnesium salt of atorvastatin having IR spectrum as shown in Figure 2.

In yet another aspect there is provided a process for the preparation of these crystalline forms of a magnesium salt of atorvastatin comprising contacting an alkali metal salt of atorvastatin salt with magnesium salt of an acid in a suitable solvent to form atorvastatin magnesium.

In another aspect there is provided a pharmaceutical composition that comprises crystalline form of atorvastatin magnesium and one or more of pharmaceutically acceptable excipients, diluents and carriers.

In another aspect there are provided the above crystalline forms of a magnesium salt of atorvastatin for inhibiting HMG-CoA reductase in a mammal. The application comprises administering, to a mammal in need thereof, a therapeutically effective amount of these crystalline forms of atorvastatin magnesium.

In another aspect there are provided the above crystalline forms of a magnesium salt of atorvastatin for treating primary hypercholesterolemia, dysbetalipoproteinemia or homozygous familial hypercholesterolemia. The application includes administering to a mammal, in need thereof, a therapeutically effective amount of the above crystalline forms of atorvastatin magnesium.

### Brief Description of the Drawings

Figure 1a is an XRD pattern showing peaks characteristic of crystalline form of magnesium salt of atorvastatin.
Figure 1b is a listing of peak values and intensities of the XRD pattern of Figure 1a.
Figure 2 is an infrared spectrum showing characteristic absorption bands of crystalline form of magnesium salt of atorvastatin.
Figure 3, for comparison, is an XRD pattern showing a halo that is characteristic of amorphous atorvastatin magnesium.

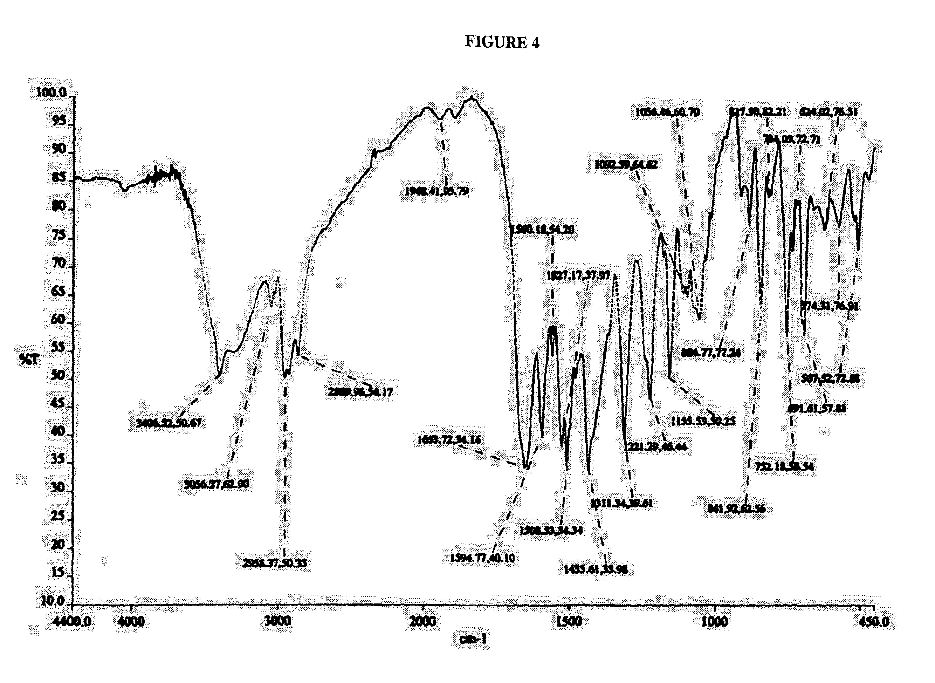

Figure 4, for comparison, is an infrared spectrum showing characteristic absorption bands of amorphous atorvastatin magnesium.

### Detailed Description of the Invention

The term "atorvastatin magnesium" as used herein refers to a salt, which includes atorvastatin anions and magnesium cations, in a crystalline form.

Further, the term "atorvastatin magnesium" also encompasses stoichiometric, as well as non-stoichiometric ratios, of atorvastatin anions and magnesium cations. The ratio of atorvastatin to magnesium is not required to be 1:1 in order to be termed atorvastatin magnesium. Atorvastatin magnesium may be formed as a salt having a 2:1 molar ratio between atorvastatin anions and magnesium cations (i.e., atorvastatin hemi-magnesium). Atorvastatin hemi-magnesium may be formed even when an excess of atorvastatin or an excess of magnesium salt of an acid is used in the salt formation.

Atorvastatin magnesium and particularly atorvastatin hemi-magnesium may exist in anhydrous, hydrated and solvated forms.

The crystalline form of magnesium salt of atorvastatin can be characterized by XRD, with a pattern which can be expressed in terms of the 20, d-spacings and relative intensities. An illustrative example is shown in Figure 1.

| **2**θ **(**± **0.2)** | **d-spacing** | **Intensity** | **I/I_{O}** |
|---|---|---|---|
| 8.600 | 10.2733 | 5346 | 61 |
| 8.800 | 10.0403 | 4882 | 55 |
| 9.660 | 9.1483 | 4493 | 51 |
| 9.880 | 8.9451 | 3591 | 41 |
| 11.340 | 7.7965 | 3405 | 39 |
| 11.520 | 7.6750 | 3131 | 36 |
| 11.800 | 7.4935 | 2832 | 32 |
| 12.080 | 7.3205 | 2808 | 32 |
| 12.200 | 7.2487 | 2813 | 32 |
| 16.140 | 5.4870 | 5228 | 59 |
| 16.760 | 5.2854 | 3179 | 36 |
| 17.960 | 4.9349 | 8817 | 100 |
| 18.480 | 4.7972 | 5997 | 68 |
| 18.620 | 4.7614 | 5777 | 66 |
| 19.940 | 4.4491 | 3855 | 44 |
| 20.320 | 4.3667 | 3605 | 41 |
| 21.260 | 4.1757 | 5888 | 67 |
| 21.440 | 4.1411 | 6280 | 71 |
| 21.600 | 4.1108 | 5955 | 68 |
| 22.460 | 3.9553 | 3572 | 41 |
| 22.860 | 3.8870 | 3684 | 42 |
| 22.980 | 3.8669 | 3864 | 44 |
| 23.120 | 3.8438 | 3678 | 42 |
| 23.720 | 3.7479 | 3357 | 38 |
| 23.960 | 3.7109 | 3336 | 38 |
| 24.200 | 3.6747 | 3107 | 35 |
| 25.200 | 3.5311 | 2418 | 27 |
| 25.460 | 3.4956 | 2319 | 26 |
| 26.720 | 3.3336 | 2484 | 28 |
| 27.440 | 3.2477 | 2476 | 28 |
| 27620 | 3.2269 | 2564 | 29 |
| 27.720 | 3.2155 | 2560 | 29 |
| 27.940 | 3.1907 | 2519 | 29 |
| 29.160 | 3.0599 | 2243 | 25 |
| 29.500 | 3.0254 | 2287 | 26 |
| 29.640 | 3.0115 | 2313 | 26 |
| 29.920 | 2.9839 | 2348 | 27 |
| 30.060 | 2.9703 | 2360 | 27 |

The crystalline form of a magnesium salt of atorvastatin can be characterized by its IR spectrum. An illustrative example of such a spectrum in potassium bromide is shown in Figure 2. Characteristic absorption bands of the crystalline form of atorvastatin magnesium are observed at 510.0, 525.0, 614.3, 692.0, 716.4, 755.9, 811.9, 847.4, 885.9, 916.9, 970.6, 1014.0,1031.7, 1076.2, 1092.0, 1110.7, 1135.4, 1222.5, 1313.0, 1435.8, 1506.1, 1524.4, 1594.4, 1654.4, 1898.2, 1947.3, 2954.3, 3283.6, 3405.8, and 36G7.3 cm⁻¹.

Also, the present invention provides processes for preparing a crystalline atorvastatin magnesium. The process comprises contacting an atorvastatin alkali metal salt with the magnesium salt of an acid in a suitable solvent to form a crystalline atorvastatin magnesium.

Atorvastatin alkali metal salts, to be used in the process, may be obtained from a compound of Formula III, for example, by using methods known in the literature. For example, they may be obtained by processes comprising contacting the compound of Formula III with an acid to hydrolyse the ketal group, followed by addition of an alkali metal hydroxide to remove the tertiary butyl group along with the formation of a metal salt.

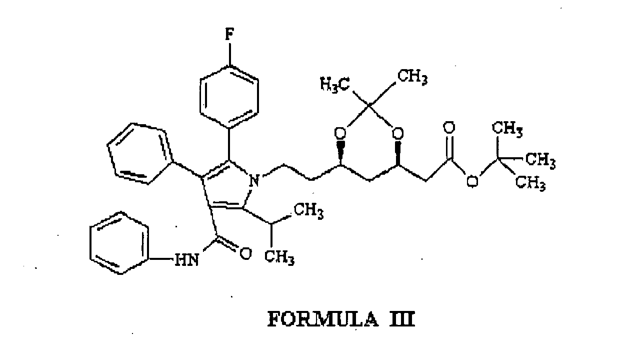

Suitable acids include mineral acids, such as hydrochloric acid. Suitable hydroxides of alkali metals include sodium hydroxide, lithium hydroxide and potassium hydroxide.

Suitable solvents for preparing atorvastatin magnesium include any solvent capable of dissolving an alkali metal atorvastatin salt and from which crystalline atorvastatin magnesium may be isolated.

Suitable solvents for carrying out the process include hydroxylic solvents, such as water, lower alkanols or mixtures thereof The hydroxylic solvents may be made acidic or basic by the addition of a mineral acid or alkali metal hydroxide if desired.

Suitable lower alkanols include primary, secondary and tertiary alcohols having one to six carbon atoms; primary, secondary and tertiary alcohols having one to four carbon atoms, for example methanol, ethanol, n-propyl alcohol, isopropyl alcohol, isobutanol, n-butanol, t-butanol and mixtures thereof.

Suitable magnesium salts of an acid to be used in the processes may be a salt of any inorganic or organic acid, such as magnesium chloride, magnesium nitrate, magnesium sulphate, magnesium phosphate, magnesium carbonate, magnesium dihydrogenphosphate, magnesium oxalate, magnesium acetate, magnesium lactate, magnesium succinate, magnesium citrate or mixtures thereof.

Once atorvastatin magnesium is crystallized, either spontaneously, upon cooling, upon seeding, or by any other inducement, the crystal may be isolated by filtration or any other conventional means known in the art. The isolated crystals are dried by conventional means.

By the way of comparison, atorvastatin magnesium in an amorphous form is also described. The amorphous atorvastatin magnesium may be characterized by its IR spectrum in potassium bromide, for example, as shown in. Figure 2. Characteristic absorption bands of amorphous atorvastatin magnesium are observed at about 507.5, 574.3, 624.0, 691.6, 734.0, 752.2, 817.9, 841.9, 884.8, 1054.5, 1092.6, 1155.5, 1221.3, 1311.3, 1435.6, 1508.5, 1527.2, 1560.2, 1594.8, 1653.7, 1948.4, 2870.0, 2958.4, 3056.3, and 3406.5 cm⁻¹.

Amorphous atorvastatin magnesium may be prepared by dissolving a crystalline form of a magnesium salt of atorvastatin in one or more suitable organic solvents and adding an anti-solvent(s) to the solution to obtain an amorphous atorvastatin magnesium.

Suitable organic solvents include one or more solvents that have the ability to dissolve crystalline atorvastatin magnesium. These solvents include, for example, tetrahydrofuran, dimethylsulphoxide, chloroform or mixtures thereof.

Any organic solvent in which atorvastatin magnesium is not soluble may be used as an anti-solvent. These anti-solvents include, for example, n-hexane, n-heptane, cyclohexane, hexane fraction, heptane fraction and mixtures thereof. For example, the solvent used for dissolving crystalline atorvastatin magnesium may be tetrahydrofuran and the anti-solvent may be cyclohexane.

The product can then be recovered by filtration at ambient temperature. The filtered material can be further dried to remove surface solvents in one or more of vacuum tray drier, tray drier, fluid bed drier or a rotary vacuum drier to obtain the amorphous atorvastatin magnesium.

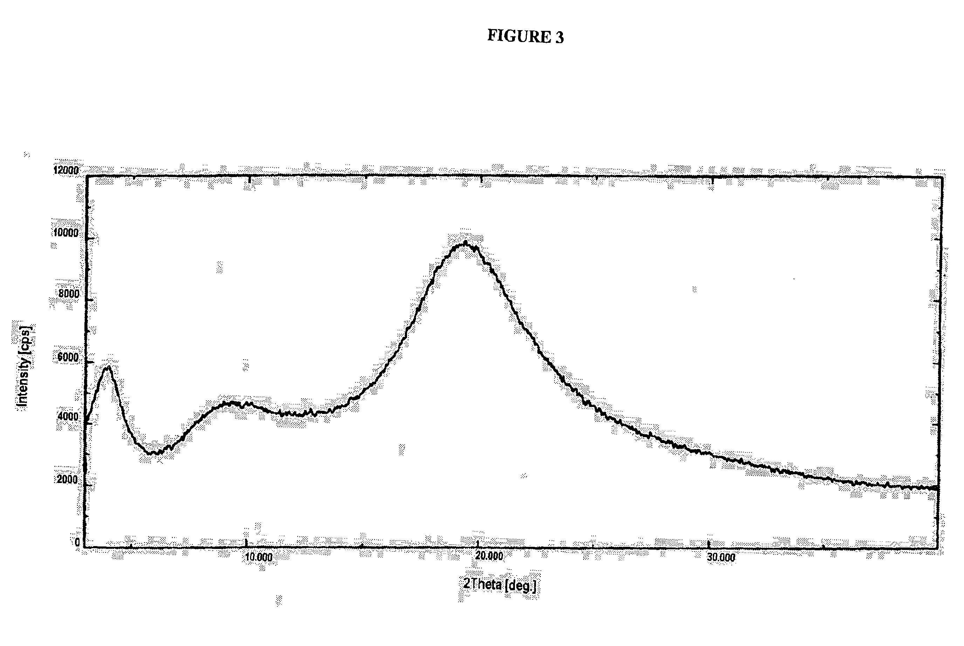

Amorphous atorvastatin magnesium may be characterized by its XRD pattern. Such an illustrative example of a spectrum is shown in Figure 3. This XRD pattern (Figure 3) shows no peaks which are characteristic of a crystalline form of atorvastatin calcium (Figure 1), thus demonstrating an amorphous nature in the product.

Also disclosed herein is another process for the preparation of amorphous atorvastatin magnesium. The process includes dissolving a crystalline form of a magnesium salt of atorvastatin in one or more solvents, and removing the solvent from the solution to obtain an amorphous atorvastatin magnesium. One or more co-solvents may be added to obtain or augment the solution.

Suitable solvents used to dissolve a crystalline form of a magnesium salt of atorvastatin are described above.

Suitable co-solvents may be those in which a magnesium salt of atorvastatin is not readily dissolved but is sparingly or moderately soluble, or the anti-solvents described above but added in quantities which are not sufficient to substantially precipitate atorvastatin magnesium. Suitable co-solvents include cyclohexane and toluene.

The process of solvent removal includes, for example, concentration, evaporation, flash evaporation, spray drying, freeze drying or lyophilization of a solution of atorvastatin magnesium.

The atorvastatin magnesium may be formulated into pharmaceutical compositions. The pharmaceutical compositions according to the invention include crystalline forms of a magnesium salt of atorvastatin as an active ingredient, along with one or more pharmaceutically acceptable excipients, diluents and/or carriers. The pharmaceutical composition may be in the form of oral, buccal, rectal, topical and parenteral (including subcutaneous, intramuscular, and ophthalmic administration) dosage forms. These dosage forms also include solid dosage forms, such as powder, tablets, capsules, suppositories, sachets, troches and lozenges, as well as liquid suspension and elixirs.

The invention provides crystalline forms of atorvastatin magnesium for the following treatment methods.

A method of inhibiting HMG-CoA reductase comprises administering to a mammal, in need thereof, a therapeutically effective amount of a crystalline form of atorvastatin magnesium.

Also disclosed is a method of treating primary hypercholesterolemia, dysbetalipoproteinemia or homozygous familial hypercholesterolemia. The method includes administering to a mammal in need thereof, a therapeutically effective amount of a crystalline and/or amorphous form of a magnesium salt of atorvastatin. The therapeutically effective amount of a crystalline and/or amorphous form of a magnesium salt of atorvastatin may be administered as a component of any of the pharmaceutical compositions described herein.

While the present invention has been described in terms of its specific embodiments certain modifications and equivalents will be apparent to those skilled in the art and are included within the scope of the present invention. The following examples illustrate various implementations of the invention without being limiting.

### EXAMPLES

### Example 1: Preparation of Crystalline Atorvastatin Magnesium

To a solution of (4R-cis)-1,1-Dimethylethyl-6-{2[2-(4-fluorophenyl)-5-(1-methylethyl)-3-phenyl-4-[(phenyl amino)-carbonyl]-1H-pyrrol-1yl]ethyl-2,2-dimethyl-1,3-dioxane-4-acetate in methanol, 1N hydrochloric acid (90.3ml) was added at 20-26°C in 15 minutes. The reaction mixture was stirred for 6 hours at the same temperature until the reaction was completed.

The pH of the resulting mixture was adjusted to about 12 by adding 10% w/v aqueous sodium hydroxide solution (107.5 ml) at 25-30°C and the resulting mixture was stirred for 6 hours at 25-30°C. The pH of the reaction mixture was monitored and maintained at about 12 throughout the course of the reaction by adding 10% w/v aqueous sodium hydroxide solution as needed. After the reaction was completed, the mass was filtered and concentrated.

Water, methanol and methyl tertiary butyl ether were added to the concentrated mass at room temperature. The reaction mixture was stirred and then allowed to settle.

The organic layer was separated and discarded. The aqueous layer was washed with methyl tertiary butyl ether (170 ml) and 6N hydrochloric acid was slowly added to adjust the pH to 7.8 to 8.0. The reaction mixture was heated to 45-55°C and an aqueous solution of magnesium acetate tetrahydrate in deionized water was slowly added at the same temperature. The reaction mixture was stirred for 1. hour at room temperature, filtered and washed with deionized water. The product was dried under vacuum (30 mm Hg) at 50-55°C, till the moisture content was about 3-7% w/w, to get 38 grams of crystalline atorvastatin magnesium.

### Example 2: Preparation of Crystalline Atorvastatin Magnesium From Atorvastatin Calcium

1N Hydrochloric acid (50ml) was added gradually to a suspension of [R-(R*, R*)]-atorvastatin calcium (50g) in deionized water (750ml) at room temperature to adjust the pH to 3.5 to 4.0. The mixture was extracted with ethyl acetate. The organic layer was washed with 20% aqueous brine (150ml) and concentrated under vacuum at 40-45°C to get the residue. To the resulting residue, methanol was added and then recovered completely under vacuum at 40-45°C twice to remove traces of ethyl acetate. Methanol (500ml) was again added to the residue at room temperature. An aqueous sodium hydroxide solution (8g in 400 ml deionized water) was added gradually to it and the mixture was stirred for 2 hours at room temperature. The pH of the reaction mixture was adjusted to 7.8 to 8.0 by adding 1N hydrochloric acid (25ml) slowly at room temperature. An aqueous solution of magnesium acetate tetrahydrate (10g in 1200ml deionized water) was added in 60 minutes at room temperature and stirred for 1 hour at room temperature. The solid separated was filtered and dried to get 45 g of crystalline atorvastatin magnesium

A suspension of atorvastatin magnesium in methanol (169 ml) and water (957 ml) was stirred for 12 hours at room temperature. The suspension was filtered and washed with a mixture of methanol (67.5 ml) and water (382.5 ml) and dried to get 44 g of pure crystalline atorvastatin magnesium.

### Example 3: Application of crystalline atorvastatin magnesium for the preparation of Amorphous Atorvastatin Magnesium

Crystalline atorvastatin magnesium (70g) was dissolved in tetrahydrofuran (182 ml) at room temperature and stirred for 30 minutes. The solution was filtered through a celite bed and the bed was washed with tetrahydrofuran (28ml). The above solution was added to cyclohexane (2100 ml taken in a separate vessel) slowly for 2 hours while stirring moderately at 22-25°C. It was then stirred vigorously for 30 minutes at 22-25°C, and the separated solid was filtered and washed with cyclohexane (70ml). The material was dried under vacuum at 60-70°C to get 66 g of the amorphous atorvastatin magnesium.

### Example 4: Application of crystalline atorvastatin magnesium for the preparation of Amorphous Atorvastatin Magnesium

Crystalline atorvastatin magnesium (70g) was dissolved in tetrahydrofuran (182ml) at room temperature and was stirred for 30 minutes. The solution was filtered through a celite bed and was washed with tetrahydrofuran (28ml). The solvent was evaporated under vacuum at 60-70°C to give 66 g of the amorphous atorvastatin magnesium.

## Claims

1. A crystalline form of a magnesium salt of atorvastatin having:
(a) an XRD pattern comprising peaks at 2Θ values of about 8.60, 16.14, 17.96 and 21.44±0.2, and/or
(b) an IR spectrum comprising absorption bands at about 510.0, 525.0, 614.3, 692.0, 716.4, 755.9, 811.9, 847.4, 885.9, 916.9, 970.6, 1014.0, 1031.7, 1076.2, 1092.0, 1110.7, 1155.4, 1222.5, 1313.0, 1435.8, 1506.1, 1524.4, 1594.4, 1654.4, 1898.2, 1947.3, 2954.3, 3283.6, 3405.8, and 3667.3 cm⁻¹.

2. The crystalline form of claim 1(a), further comprising characteristic peaks at 2θ values of about 9.66 and 11.34 ± 0.2.

3. The crystalline form of claim 1(a), further comprising characteristic peaks at 2θ values 8.80, 18.48, 18.62, 21.26, and 21.60 ± 0.2.

4. A process for the preparation of crystalline magnesium salt of atorvastatin according to any preceding claim, comprising contacting an alkali metal salt of atorvastatin with a magnesium salt of an acid in the presence of a solvent to form a crystalline atorvastatin magnesium.

5. The process of claim 4, wherein the alkali metal salt comprises one or more of atorvastatin potassium, atorvastatin sodium and atorvastatin lithium.

6. The process of claim 4, wherein the magnesium salt of an acid comprises a magnesium salt of any inorganic or organic acid.

7. The process of claim 4, wherein the magnesium salt of an acid comprises one or more of magnesium chloride, magnesium nitrate, magnesium sulphate, magnesium phosphate, magnesium carbonate, magnesium dihydrogenphosphate, magnesium oxalate, magnesium acetate, magnesium lactate, magnesium succinate, and magnesium citrate.

8. The process of claim 4, wherein the solvent comprises one or more hydroxylic solvents.

9. The process of claim 12, wherein the hydroxylic solvents comprise water, lower alkanols and mixtures thereof.

10. The process of claim 9, wherein the lower alkanols comprise primary, secondary and tertiary alcohols having one to six carbon atoms.

11. The process of claim 9, wherein the lower alkanols comprise primary, secondary and tertiary alcohols having one to four carbon atoms.

12. The process of claim 11, wherein the lower alkanols comprise one or more of methanol, ethanol, n-propyl alcohol, isopropyl alcohols, isobutanol, n-butanol, and t-butanol.

13. A pharmaceutical composition comprising crystalline atorvastatin magnesium of claim 1. and one or more of pharmaceutically acceptable excipients, diluents and carriers.

14. The crystalline form of a magnesium salt of atorvastatin of claim 1 for treating primary hypercholesterolemia, dysbetalipoproteinemia or homozygous familial hypercholesterolemia in a mammal.

15. The crystalline form of a magnesium salt of atorvastatin of claim 1 for inhibiting HMG-CoA reductase in a mammal.

## Patentansprüche

1. Kristalline Form eines Magnesiumsalzes von Atorvastatin mit
(a) einem Röntgenbeugungsdiagramm mit Maxima bei 2θ-Werten von etwa 8,60, 16,14, 17,96 und 21,44+0,2 und/oder
(b) einem IR-Spektrum mit Absorptionsbanden bei etwa 510,0, 525,0, 614,3, 692,0, 716,4, 755,9, 811,9, 847,4, 885,9, 916,9, 970,6, 1014,0, 1031,7, 1076,2, 1092,0, 1110,7, 1155,4, 1222,5, 1313,0, 1435,8, 1506,1, 1524,4, 1594,4, 1654,4, 1898,2, 1947,3, 2954,3, 3283,6, 3405,8 und 3667,3 cm⁻¹.

2. Kristalline Form nach Anspruch 1(a), ferner charakteristische Maxima bei 2θ-Werten von etwa 9,66 und 11,34+0,2 umfassend.

3. Kristalline Form nach Anspruch 1(a), ferner charakteristische Maxima bei 2θ-Werten von etwa 8,80, 18,48, 18,62, 21,26 und 21,60±0,2 umfassend.

4. Verfahren zur Herstellung von kristallinem Magnesiumsalz von Atorvastatin nach einem der vorangehenden Ansprüche, umfassend Inkontaktbringen eines Alkalimetallsalzes von Atorvastatin mit einem Magnesiumsalz einer Säure in Gegenwart eines Lösungsmittels zur Bildung eines kristallinen Magnesiumatorvastatins.

5. Verfahren nach Anspruch 4, wobei das Alkalimetallsalz eines oder mehrere von Kalium-, Natrium- und Lithiumatorvastatin umfasst.

6. Verfahren nach Anspruch 4, wobei das Magnesiumsalz einer Säure ein Magnesiumsalz einer beliebigen anorganischen oder organischen Säure umfasst.

7. Verfahren nach Anspruch 4, wobei das Magnesiumsalz einer Säure eines oder mehrere von Magnesiumchlorid, Magnesiumnitrat, Magnesiumsulfat, Magnesiumphosphat, Magnesiumcarbonat, Magnesiumdihydrogenphosphat, Magnesiumoxalat, Magnesiumacetat, Magnesiumlaktat, Magnesiumsuccinat und Magnesiumzitrat umfasst.

8. Verfahren nach Anspruch 4, wobei das Lösungsmittel eines oder mehrere Hydroxyllösungsmittel umfasst.

9. Verfahren nach Anspruch 8, wobei die Hydroxyllösungsmittel Wasser, niedrige Alkanole und deren Mischungen umfassen.

10. Verfahren nach Anspruch 9, worin die niedrigen Alkanole primäre, sekundäre und tertiäre Alkohole mit einem bis sechs Kohlenstoffatomen umfassen.

11. Verfahren nach Anspruch 9, wobei die niedrigen Alkanole primäre, sekundäre und tertiäre Alkohole mit einem bis vier Kohlenstoffatomen umfassen.

12. Verfahren nach Anspruch 11, wobei die niedrigen Alkanole eines oder mehrere von Methanol, Ethanol, n-Propanol, Isopropanol, Isobutanol, n-Butanol und t-Butanol umfassen.

13. Pharmazeutische Zusammensetzung, umfassend kristallines Magnesiumatorvastatin nach Anspruch 1 und einen oder mehrere von pharmazeutisch zulässigen Hilfsstoffen, Verdünnern und Trägern.

14. Kristalline Form eines Magnesiumsalzes von Atorvastatin nach Anspruch 1 zur Behandlung von primärer Hypercholesterinämie, Dysbetalipoproteinämie oder homozygoter familiärer Hypercholesterinämie bei einem Säugetier.

15. Kristalline Form eines Magnesiumsalzes von Atorvastatin nach Anspruch 1 zur Inhibierung von HMG-CoA-Reduktase bei einem Säugetier.

## Revendications

1. Forme cristalline d'un sel de magnésium d'atorvastatine présentant:
(a) un motif de diffraction des rayons X (XRD) comprenant des pics à des valeurs 2θ d'environ 8,60, 16,14, 17,96 et 21,44 ± 0,2, et/ou
(b) un spectre IR comprenant des bandes d'absorption à environ 510,0, 525,0, 614,3, 692,0, 716,4, 755,9, 811,9, 847,4, 885,9, 916,9, 970,6, 1014,0, 1031,7, 1076,2, 1092,0, 1110,7, 1155,4, 1222,5, 1313,0, 1435,8, 1506,1, 1524,4, 1594,4, 1654,4, 1898,2, 1947,3, 2954,3, 3283,6, 3405,8 et 3667,3 cm-1.

2. Forme cristalline selon la revendication 1(a), comprenant, en outre, des pics caractéristiques à des valeurs 2θ d'environ 9,66 à 11,34 ± 0,2.

3. Forme cristalline selon la revendication 1(a), comprenant en outre des pics caractéristiques à des valeurs 2θ de 8,80, 18,48, 18,62, 21,26 et 21,60 ± 0,2.

4. Procédé pour la préparation d'un sel de magnésium cristallin d'atorvastatine selon l'une quelconque des revendications précédentes, comprenant la mise en contact d'un sel de métal alcalin d'atorvastatine avec un sel de magnésium d'un acide en présence d'un solvant de manière à former un magnésium d'atorvastatine cristallin.

5. Procédé selon la revendication 4, dans lequel le sel de métal alcalin comprend un ou plusieurs des composés de potassium d'atorvastatine, de sodium d'atorvastatine et de lithium d'atorvastatine.

6. Procédé selon la revendication 4, dans lequel le sel de magnésium d'un acide comprend un sel de magnésium de n'importe quel acide inorganique ou organique.

7. Procédé selon la revendication 4, dans lequel le sel de magnésium d'un acide comprend un ou plusieurs des composés choisis parmi le chlorure de magnésium, le nitrate de magnésium, le sulfate de magnésium, le phosphate de magnésium, le carbonate de magnésium, le dihydrogénophosphate de magnésium, l'oxalate de magnésium, l'acétate de magnésium, le lactate de magnésium, le succinate de magnésium et le citrate de magnésium.

8. Procédé selon la revendication 4, dans lequel le solvant comprend un ou plusieurs solvants hydroxyliques.

9. Procédé selon la revendication 12, dans lequel les solvants hydroxyliques comprennent l'eau, des alcanols inférieurs et leurs mélanges.

10. Procédé selon la revendication 9, dans lequel les alcanols inférieurs comprennent des alcools primaires, secondaires et tertiaires ayant un à six atomes de carbone.

11. Procédé selon la revendication 9, dans lequel les alcanols inférieurs comprennent des alcools primaires, secondaires et tertiaires ayant un à quatre atomes de carbone.

12. Procédé selon la revendication 11, dans lequel les alcanols inférieurs comprennent un ou plusieurs des composés choisis parmi le méthanol, l'éthanol, l'alcool de n-propyle, l'alcool d'isopropyle, l'isobutanol, le n-butanol et le t-butanol.

13. Composition pharmaceutique comprenant le magnésium d'atorvastatine cristallin selon la revendication 1 et un ou plusieurs excipients diluants et véhicules acceptables au plan pharmaceutique.

14. Forme cristalline d'un sel de magnésium d'atorvastatine selon la revendication 1, pour le traitement de l'hypercholestérolémie primaire, de la dysbêtalipoprotéinémie ou de l'hypercholestérolémie familiale homozygote chez un mammifère.

15. Forme cristalline d'un sel de magnésium d'atorvastatine selon la revendication 1, pour inhiber une HMG-CoA réductase chez un mammifère.
